(19) **Europäisches Patentamt**

**European Patent Office**

**Office européen des brevets**

(11) **EP 1 452 867 B1**

(12) **EUROPEAN PATENT SPECIFICATION**

(45) Date of publication and mention
of the grant of the patent:
**07.06.2006 Bulletin 2006/23**

(51) Int Cl.:
*G01N 33/543* (2006.01)          *G01N 27/22* (2006.01)
*G01R 27/26* (2006.01)

(21) Application number: **03251110.7**

(22) Date of filing: **25.02.2003**

(54) **DNA specific capacity affinity sensor**

DNA-spezifische Kapazitiver Affinitätssensor

Capteur d'affinité de capacité spécifique à l'ADN

(84) Designated Contracting States:
**DE FR GB**

(43) Date of publication of application:
**01.09.2004 Bulletin 2004/36**

(73) Proprietor: **Hitachi, Ltd.**
**Chiyoda-ku,**
**Tokyo 101-8010 (JP)**

(72) Inventors:
• **Nakazato,Kazuo**
  **Cambridge,CB2 2RP (GB)**
• **Mizuta,Hiroshi**
  **Great Shelford, Cambridge CB2 5JW (GB)**
• **Kamahori,Masao**
  **Kokubunji-shi,**
  **Tokyo 1850-0014 (JP)**

• **Okano,Kazunori**
  **Shiki-shi,**
  **Saitama Prefecture 253-0004 (JP)**

(74) Representative: **Read, Matthew Charles et al**
**Venner Shipley LLP**
**20 Little Britain**
**London EC1A 7DH (GB)**

(56) References cited:
**US-A- 4 149 231          US-A- 5 164 319**
**US-A- 5 270 663          US-A- 5 532 128**

• **BERNEY H ET AL: "A DNA diagnostic biosensor: development, characterisation and performance" SENSORS AND ACTUATORS B, ELSEVIER SEQUOIA S.A., LAUSANNE, CH, vol. 68, no. 1-3, 25 August 2000 (2000-08-25), pages 100-108, XP004216599 ISSN: 0925-4005**

EP 1 452 867 B1

**Description**

This invention relates to sensor for sensing capacitance and has particular but not exclusive application to use as a biosensor for direct detection of DNA sequences based on a capacitance measurement.

Hitherto, biosensors have been proposed for the direction of DNA sequences through capacitance measurement. For example, a biosensor is described in "A Biosensor For Direct Detection Of DNA Sequences Based On Capacitance Measurements" C. Guiducci et al, ESSDERC 2002 pp 479-482. The biosensor comprises two glass slides with gold disc electrodes thereon spaced by a distance of 2nm. A fluid containing DNA is provided as a dielectric between the two electrodes. The capacitance of the arrangement is tested by applying pulses of different frequencies between the electrodes and monitoring the current flowing between them. The capacitance of the cell can be determined from the current measurements by a linear regression technique.

The capacitance of the cell changes as a result of the DNA present in the fluid between the electrodes. One of the electrodes can be functionalised to detect a particular DNA strand. The functionalisation is carried by causing strands of a known DNA sequence to attach themselves at one end to the gold electrode. When a fluid is introduced between the electrode plates containing a complimentary DNA strands, they bind to the strands already attached to the electrode and the electrical capacitance between the two gold electrodes is changed as a result. A disadvantage of the arrangement is that a number of measurements need to be taken at different frequencies and a linear regression technique is required in order to determine the capacitance of the cell. Also, parasitic capacitances give rise to measurement errors when measuring very small capacitive changes resulting from the presence or absence of particular DNA strands.

US-A-5,164,319 discloses a pixelated capacitor sensor array driven by an oscillator to detect the capacitance by the pixels individually. Reference is also directed to US-A-5,532,128 and US 4,149,231.

The present invention provides an alternative technique for measuring capacitive changes in a capacitive sensor.

According to the invention there is provided a sensor comprising an array of capacitive elements configured to form a capacitive coupling as a function of a medium to be sensed, a plurality of sensor circuit units associated with the capacitive elements, the circuit units each exhibiting a respective time constant as a function of the capacitive coupling of their associated capacitive element, the sensor circuit units being interconnected in series in a circuit that oscillates with a frequency dependent on the time constants of the circuit units, and frequency detection circuitry to detect the frequency of oscillation of the circuit.

The circuit units may comprise a plurality of inverters and the oscillatory circuit may comprise a ring oscillator.

The medium to be sensed may comprise a body that forms a capacitive coupling with the capacitive elements, enabling the sensor to detect the presence of the object.

Alternatively, the capacitive elements may be configured for forming a capacitive coupling with a fluid containing material to be sensed, e.g. for use as a biosensor to detect DNA fragments with a particular molecular structure. The conductive members can be functionalised for detecting predetermined DNA targets.

A capacitor plate may be spaced from the conductive members to provide a region to receive the fluid or the conductive members may be configured to form a mutual capacitive coupling with one another for sensing characteristics of the fluid.

The conductive members may comprise plates that are formed on a substrate together with the circuit units and the sensor may include wells to receive a fluid to be sensed, with the conductive members extending into the wells.

The sensor may include selecting circuitry to select the circuit units individually such that the frequency characteristic of the oscillatory circuit is primarily dependent on the capacitive coupling of the or each said selected circuit unit.

The circuit units may comprise inverters with a time constant that is a function of their capacitive coupling and the inverters may include an input to alter their time constant selectively. The inverters may comprise complementary transistors.

The invention also includes a method of operating the sensor including placing a fluid based medium to be sensed in contact with the capacitive elements and monitoring the frequency of operation of the oscillatory circuit.

In order that the invention may be more fully understood embodiments thereof will now be described by way of example with reference to the accompanying drawings in which:

Figure 1 is a schematic perspective view of capacitive elements of a sensor in accordance with the invention;
Figure 2 is a schematic sectional view of a sensor according to the invention, for use as a capacitive proximity detector;
Figure 3 is a schematic sectional view of a sensor according to the invention, for detecting a fluid medium such as a liquid containing a DNA sample;
Figure 4 is a circuit diagram of a ring oscillator used in the sensor;
Figure 5 is a circuit diagram of a CMOS inverter used in the circuit units shown in Figure 4;
Figure 6 is a schematic plan view of an implementation of one of the circuit units shown in Figure 4
Figure 7 is a sectional view of the circuit unit shown in Figure 6;

Figure 8 is a circuit diagram of an alternative IIL inverter for use the circuit units shown in Figure 4;

Figure 9 is a circuit diagram of a ring oscillator used in the sensor, in which the circuit units are individually addressable;

Figure 10 is a circuit diagram of a CMOS inverter used in the circuit units shown in Figure 9;

Figure 11 is a plan view of an alternative embodiment of sensor according to the invention with wells to receive a fluid to be sensed;

Figure 12 is a schematic sectional view of the sensor shown in Figure 11;

Figure 13 is a circuit diagram of a ring oscillator used in the sensor of Figures 11 and 12; and

Figure 14 illustrates modifications to the capacitive plates of Figures 1 and 11 and modifications thereto to reduce DC leakage current.

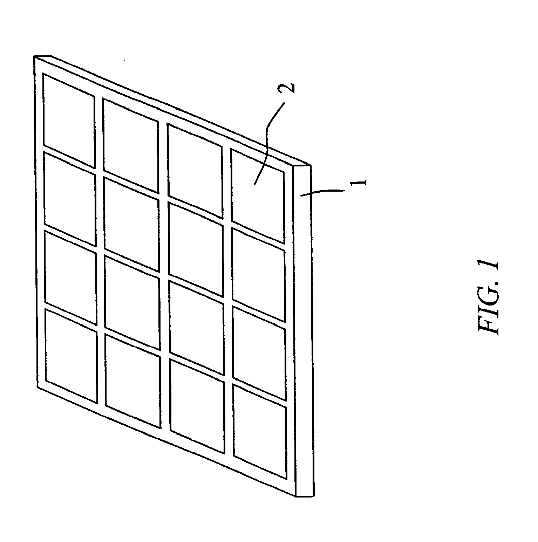

Figure 1 illustrates schematically a semiconductor substrate 1 on which an array of conductive members in the form of plates 2 are formed to act as capacitive sensor elements. The conductive plates 2 may comprise regions of gold or other materials deposited and patterned by conventional techniques.

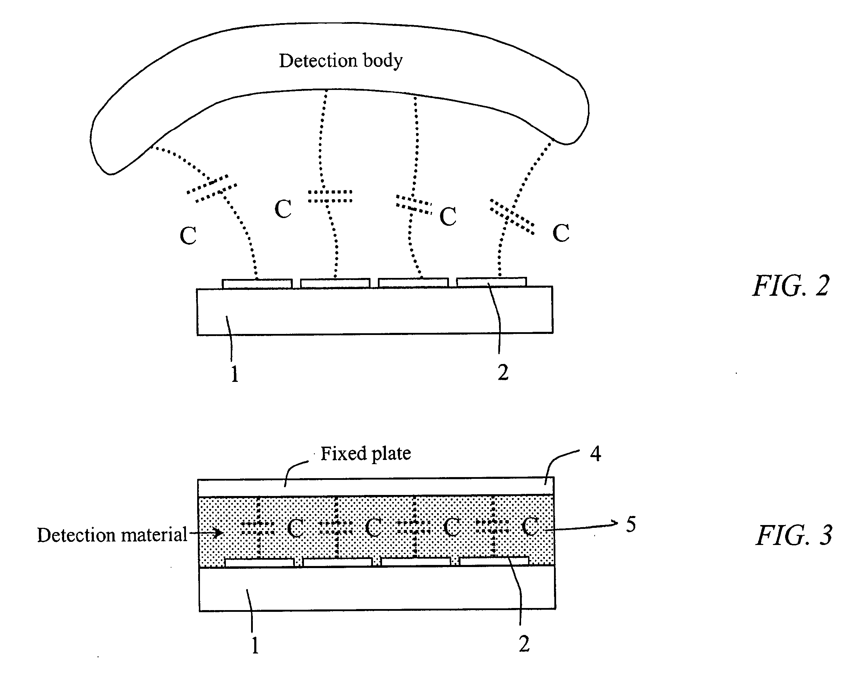

Figure 2 illustrates the manner in which the individual plates 2 may form capacitive couplings C with an adjacent body 3 to be detected.

Figure 3 illustrates how the plates 2 may be used in a biosensor in which a medium 5 to be sensed, that may include DNA strands, passes between the substrate 1 and a fixed conductive plate 4 so as to form individual capacitive couplings C dependent upon the characteristics of medium 5 between the substrate 1 and the fixed plate 4.

The electrode plates 2 may be functionalised in the manner described by Guiducci et al *supra.* In this way, the material 5 may include DNA strands of a complimentary nature to the strands used to functionalise the electrodes 2 and as a result, the resulting cell shown in Figure 3 exhibits a change in capacitance when DNA that is complimentary to the DNA of the functionalised plates 2, is passed through it.

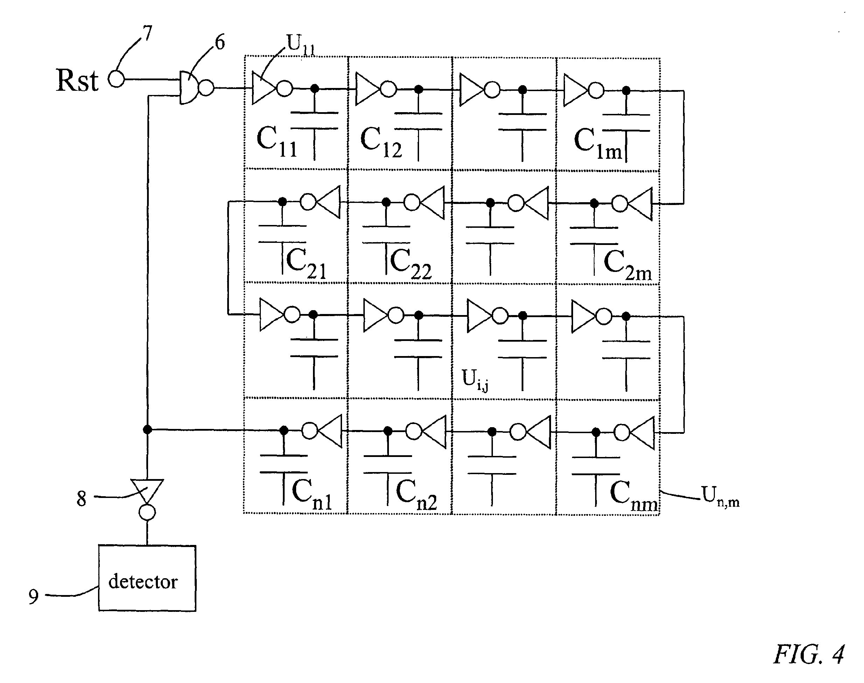

Figure 4 illustrates an electrical circuit that is used to detect the capacitive couplings C shown in Figure 2 and 3. The sensor circuit includes circuit units $U_{11}$ - $U_{nm}$ associated with the nxm array of conductive plates 2 shown in Figure 1. Each of the conductive plates 2 develops an associated capacitance in the nxm array so that for circuit $U_{11}$, the corresponding plate 2 exhibits a capacitance $C_{11}$ etc.

Each of the circuit units $U_{ij}$ includes an individual inverter $I_{ij}$. The circuit units $U_{11}$ ... $U_{nm}$ are connected in series to form a ring oscillator through a NAND gate 6 which also acts as an inverter, providing an odd number of inverter stages in the ring oscillator in order to promote oscillation of the circuit. The NAND gate 6 has a reset input 7 to allow the ring oscillator to be reset.

An output is taken from the ring oscillator through a buffer inverter 8 and fed to an output terminal with an associated frequency measuring device 9 which measures the frequency of oscillation of the ring oscillator. The buffer inverter 8 amplifies the signal of the ring oscillator and reduces the influence of large parasitic capacitances on the output terminal.

The ring oscillator has a period of oscillation T that is determined by the sum of the rise and fall times of each inverter $I_{ij}$

$$T = \sum (r_r + r_f)$$

Where rr is the rise time and $r_f$ is the fall time constants of each inverter $I_{ij}$. The rise and fall time constants $r_r$ and $r_f$, respectively, are a function of the capacitance on the output node of the inverter $I_{ij}$, which is determined by the capacitance $C_{ij}$. Thus, the aggregate capacitive coupling $\Sigma\, C_{ij}$ can be determined from the oscillation period T of the ring oscillator and so the frequency f =1/T measured by the frequency measuring device 9 provides a measurement of the capacitance.

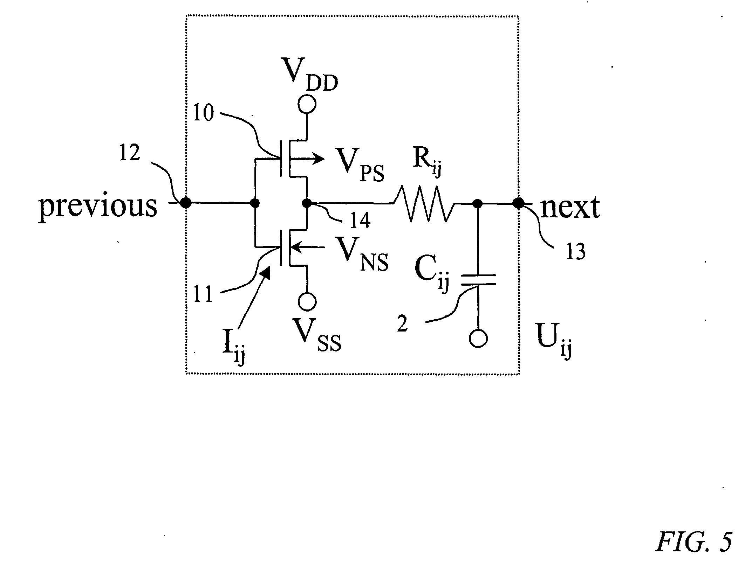

Figure 5 is a schematic circuit diagram of one of the circuit element $U_{ij}$, in which the inverter $I_{ij}$ comprises a CMOS inverter, with p and n-type MOSFETs 10, 11 connected with their source drain paths in series between voltage rails $V_{DD}$ and Vss. The circuit element $U_{ij}$ has an input node 12 and an output node 13. Input node 12 is connected to the corresponding output node of circuit unit $U_{i',j'}$ whereas the output node 13 is connected to the corresponding input node of circuit unit $U_{i'',j''}$. The capacitance $C_{ij}$ associated with the corresponding conductive plate 2 is illustrated connected to output 14 of the inverter through an associated resistance $R_{ij}$ The resistance $R_{ij}$ need not be a discrete component but an associated parasitic resistance for the circuit element.

The time delay $t_{ij}$ of the circuit unit $U_{ij}$ is given approximately by

$$.t_{ij} \sim C_{ij}(R_{ij} + \Delta V / I_{ON}(pMOSFET) + \Delta V / I_{ON}(nMOSFET))$$

where $\Delta V$ is the voltage difference between $V_{DD}$ and $V_{SS}$ and $I_{ON}(pMOSFET)$ and $I_{ON}(nMOSFET)$ are the ON currents of pMOSFET 10 and nMOSFET 11 respectively. The oscillation frequency f of the ring oscillator is given by

$$f = 1/T$$

and the oscillation period T is given by

$$T = \Sigma \, t_{ij}$$

Bias voltages $V_{PS}$ and $V_{NS}$ can be applied to the channels of the transistors 10, 11 respectively for controlling their operation as explained in more detail later.

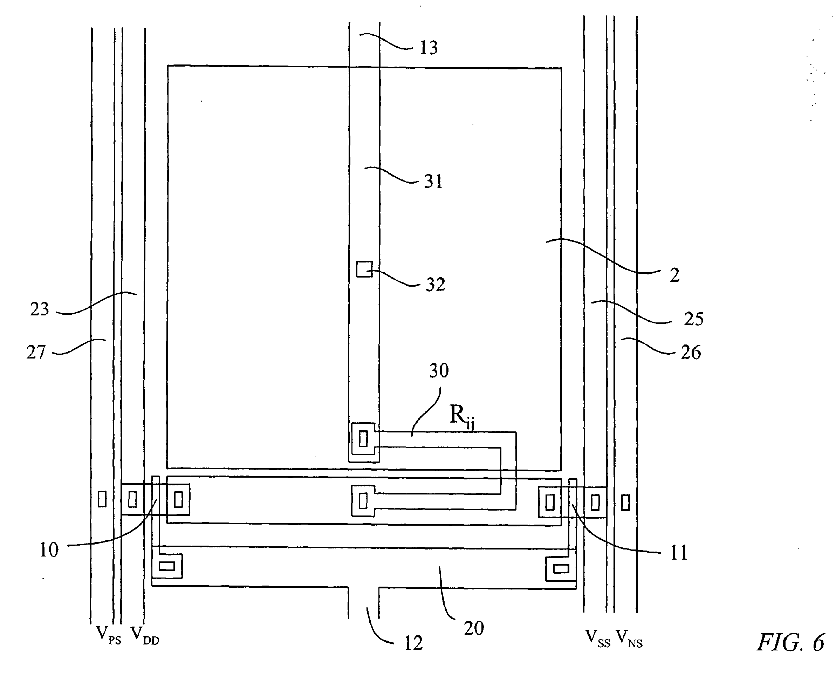

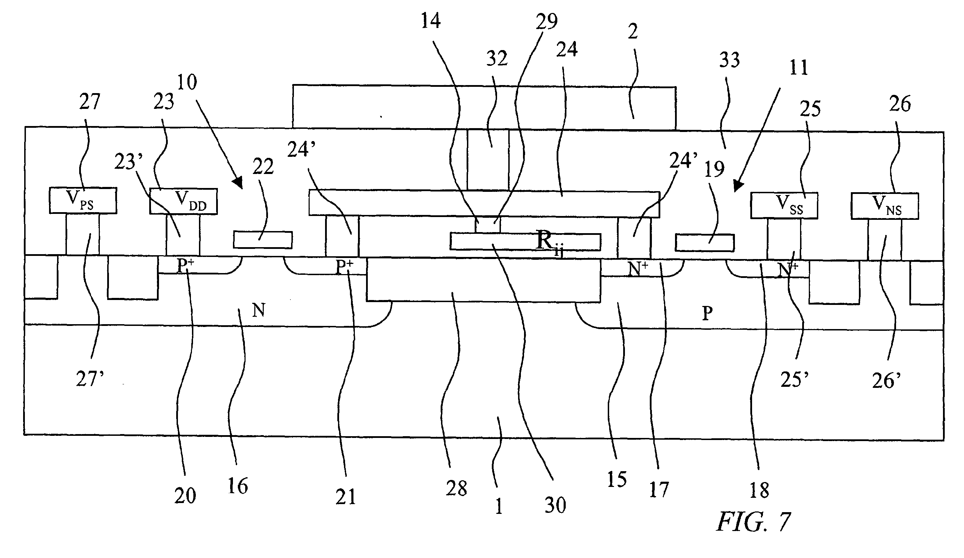

Figures 6 and 7 illustrate schematic plan and sectional views of the circuit element $U_{ij}$. Referring initially to Figure 7, the substrate 1 is formed with lightly doped p and n-type regions 15, 16 into which the complimentary transistors 10 and 11 are formed respectively. The transistor 11 includes $n^+$ source and drain regions 17, 18 and a gate 19. Transistor 10 comprises source and drain regions 20, 21 and a gate 22.

The source and drains of the transistors 10, 11 are connected in series between supply rail $V_{DD}$ provided on conductor 23, through conductor 24, to the source rail Vss provided on conductor 25. Additional rails 27, 26 are arranged to apply the bias voltages $V_{PS}$ and $V_{NS}$ to the complimentary transistors 10, 11 respectively. An insulating oxide region 28 separates the two transistor in the substrate 1.

Each of the conductive tracks 23, 24, 25, 26 and 27 are connected to their respective doped regions of the transistors 10, 11 by conductive vias 23'-27' which may be formed of polysilicon or metal (Al, AlSi, W, Ti etc).

A conductive via 29 provides a connection from the region 14 of conductive track 24 to a further conductor 30, which may be formed of polysilicon, that gives rise to the resistance $R_{ij}$. As shown in Figure 6, the conductor 30 provides a connection to a further conductive track 31 that extends to the output node 13. Conductive via 32 connects the overlying conductive plate 2 to the conductive track 31.

Referring to Figure 7, the various conductive tracks 23-28 are formed within a region of insulating silicon dioxide 33. In practice, the region 33 is formed as multiple oxide layers during an appropriate series of patterning and photolithography steps as will be readily apparent to those skilled in the art of CMOS fabrication techniques.

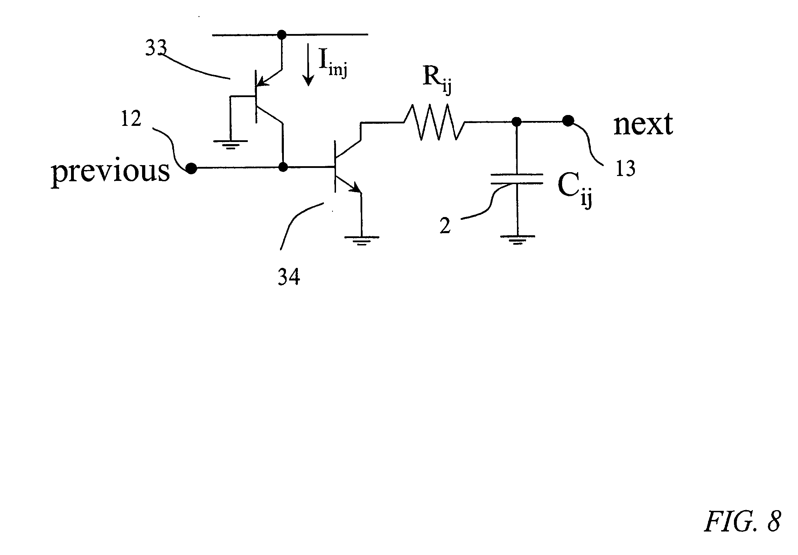

Figure 8 illustrates an alternative structure of the circuit unit $U_{ij}$ in which bipolar transistors 33, 34 are used to form the inverter $I_{ij}$. The inverter operates generally in the same way as the CMOS inverter of Figure 5 and like parts are given the same reference numbers. It will be understood that the PNP and NPN transistors 33, 34 comprise an IIL (Integrated Injection Logic) inverter. The advantage of an IIL inverter is that it can operate over a wider frequency range than CMOS devices. If the resistance $R_{ij}$ is lower than $\Delta V/I_{inj}$, where $I_{inj}$ is the injection current for the inverter, the delay of the inverter is given by

$$t_{ij} \sim C_{ij} \, \Delta V/I_{inj} \qquad\qquad\qquad (3)$$

therefore, the delay time and consequently the oscillation frequency can be widely changed by selecting the appropriate value of $I_{inj}$.

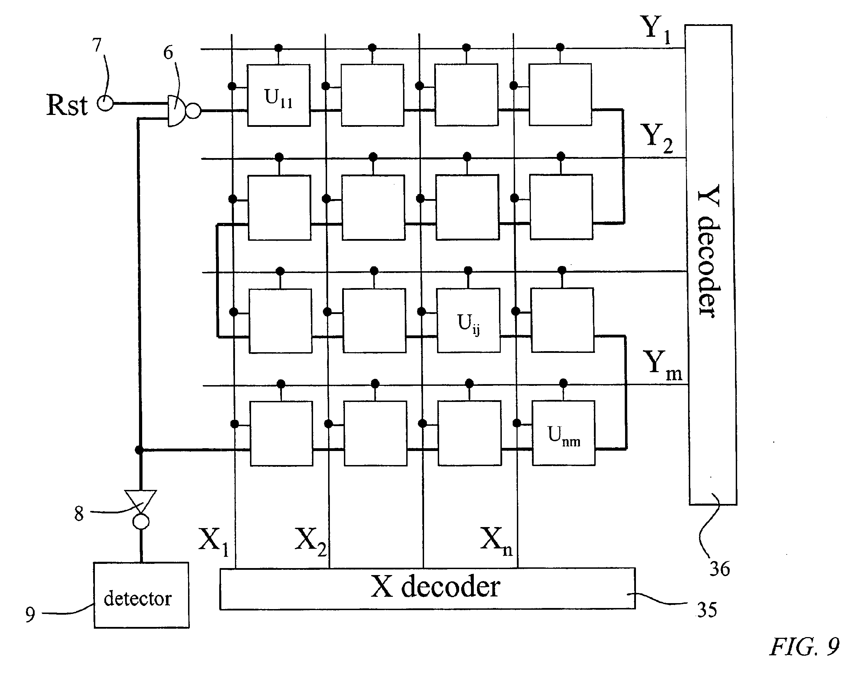

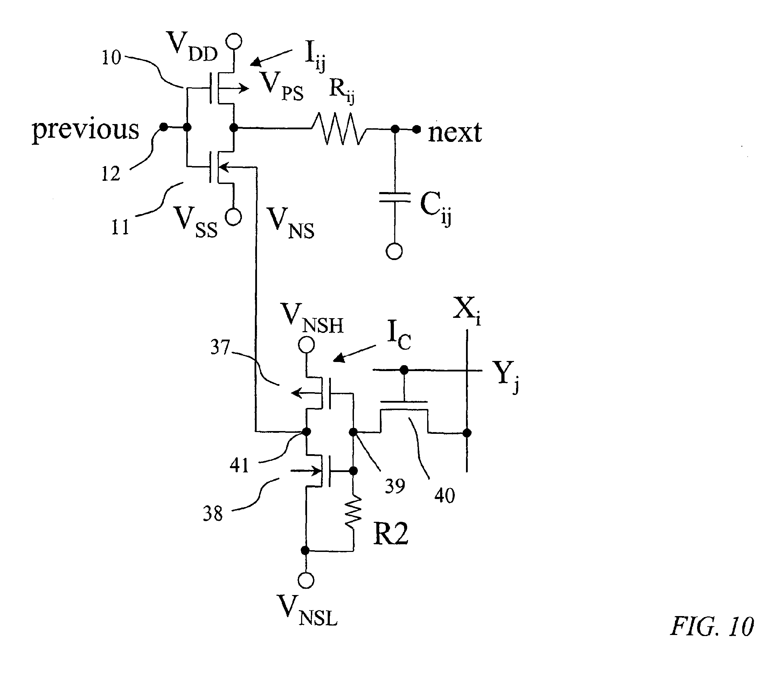

According to an advantageous feature of the invention, the capacitance $C_{ij}$ associated with each conductive plate 2 in the array can optionally be detected individually, as will now be explained with reference to Figures 9 and 10. The circuit configuration of Figure 9 is generally similar to the circuit shown in Figure 4, in which individual circuit units are arranged in an array corresponding to the conductive plates 2 (not shown in Figure 9) and the circuit units are connected in a ring oscillator including NAND gate 6, buffer amplifier 5 and frequency measuring device 9. Additionally, the circuit of Figure 9 includes row and column decoders 35, 36 which allow row and column selection signals X1...Xn; Y1...Ym to be applied to the circuit units $U_{ij}$ in order to select them individually.

Figure 10 illustrates one of the circuit units $U_{ij}$ of Figure 9. The circuit unit includes p and n MOSFET transistors 10, 11 arranged as an inverter $I_{ij}$ together with resistance $R_{ij}$ and capacitance $C_{ij}$ from the associated conductive plate 2. This configuration corresponds to the circuit of Figure 5. Additionally, the circuit unit of Figure 10 includes a CMOS inverter $I_C$ comprising pMOSFET 37 and nMOSFET 38. Input 39 to the inverter $I_C$ is controlled by MOSFET 40 which has its gate connected to the row line $Y_j$ and its source/drain path connected to the column line $X_i$. The inverter $I_C$ is connected between supply rails $V_{NSH}$ and $V_{NSL}$. Input 39 to the inverter $I_C$ is connected to the relatively low supply rail $V_{NSL}$ through resistor R2. Output 41 of the inverter $I_C$ is connected to the substrate voltage supply rail $V_{NS}$ for the nMOSFET 11 of the inverter $I_{ij}$.

Usually, the transfer MOSFET 40 is an off state and the resistor R2 keeps the gates of pMOSFET 37 and nMOSFET 38 at a low level, so that the nMOSFET substrate voltage $V_{NS}$ is maintained at a high voltage $V_{NSH}$. As a result, the ON current of nMOSFET 12 is relatively high.

The value of the capacitance Cij associated with circuit unit Uij can be selected for measurement by applying a high bias voltage on column line $X_i$ and a row voltage on line $Y_j$ so as to switch on transistor 40. This results in the gates of pMOSFET 37 and nMOSFET 38 being biased to a high level and so the substrate voltage $V_{NS}$ provided at the output of inverter $I_C$ changes to a relatively low level $V_{NSL}$, which reduces the ON current of nMOSFET 11 to a relatively low level.

From equation (2) it can be shown that the frequency f of the ring oscillator is approximately given by

$$f \sim I/\Sigma(C_{ij}\Delta V/I_{ON}) \tag{4}$$

i.e

$$f \sim I/\Sigma\ t_{ij} \tag{5}$$

where $t_{ij} = C_{ij}\ \Delta V/I_{ON}$.

Considering the individual circuit unit $U_{ij}$, when the corresponding row and column lines $X_i$, $Y_j$ are enabled, the value of $I_{ON}$ is relatively low. As a result, the corresponding value of $t_{ij}$ is relatively high as compared with the value that occurs when the row and column lines $X_i$, $Y_j$ are not enabled.

Also, for the other non-selected circuit units U in the array, their corresponding value of $I_{ON}$ is relatively high and as a result their corresponding value of $t$ is relatively low as compared with the selected cell $t_{ij}$.

From equation (4) it can be seen that the frequency of oscillation of the ring oscillator is determined primarily by the high value of $t_{ij}$ for the selected circuit i.e. the circuit unit that receives row and column enabling signals $X_i$, $Y_j$. The values of t for the non-selected circuit units are much smaller and do not contribute much to the frequency of oscillation of the ring oscillator. In this way, the capacitance of individual plates 2 can be analysed within the array by selecting particular circuit units. They can be selected individually or in particular groups, by applying appropriate enabling signals from the X and Y decoder circuits 35, 36 shown in Figure 9.

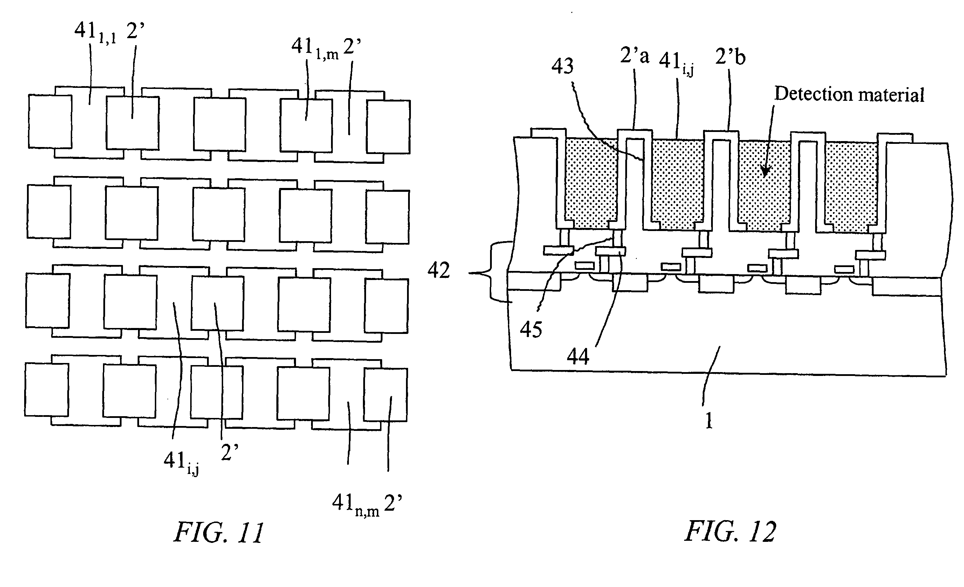

Many modifications and variations of the described sensor are possible. Figures 11 and 12 illustrate a modification in which conductive plates 2' are arranged along side walls of an array of wells $41_{ij}$. As shown in Figure 12, the structure of the device in section is generally similar to that shown in Figure 7, with substrate 1 containing a layer region 42 that contains transistors to provide inverters for the circuit units $U_{ij}$. The layer 33 is made thicker than that shown in Figure 7 and the wells 41 are formed by photolithography and etching in a conventional manner. The conductive plates 2' may be formed by sputtering or other suitable techniques along side walls 43 of the wells 41. The plates 2' are connected to the transistors by suitable vias and rails 44, 45.

Each of the electrode plates 2' extends into two adjacent wells and each circuit unit is configured to have a time constant dependant on the capacitance between the two conductive plates in each of the wells e.g. plates 2'a and 2'b in well $41_{ij}$ shown in Figure 12.

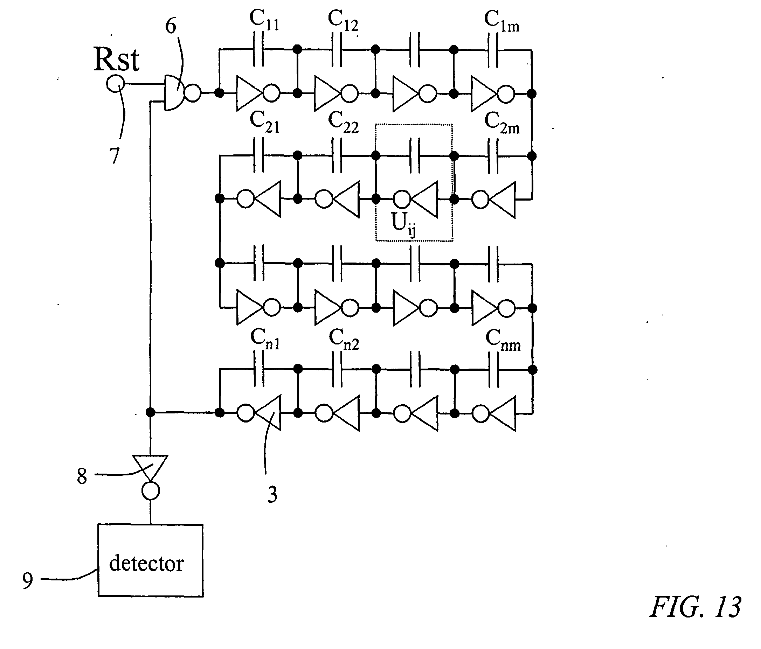

Each circuit unit $U_{ij}$ includes an inverter $I_{ij}$ formed in the layer region 42 of Figure 12 with a circuit as previously described with reference to Figure 5, together with an associated parallel capacitance $C_{ij}$ which is determined by the capacitance of an associated one of the wells 41 measured between the two conductive plates 2' within it. It will be appreciated that the capacitance $C_{ij}$ determines the time constant for the circuit unit $U_{ij}$ and as a result, the frequency of oscillation of the ring oscillator shown in Figure 13 is a function of the aggregate of the time constants of the circuit units $U_{ij}$. It will be understood that the circuit of Figure 13 can be modified to include addressing circuitry as described with reference to Figure 10 in order to select the wells 41 individually.

The advantage of providing the wells 41 is that the surface of the device is open as compared with Figure 3, making it easy to supply the medium to be detected. Furthermore, since no fixed plate is required the risk of frequency reading being degraded by spurious external capacitances, is reduced.

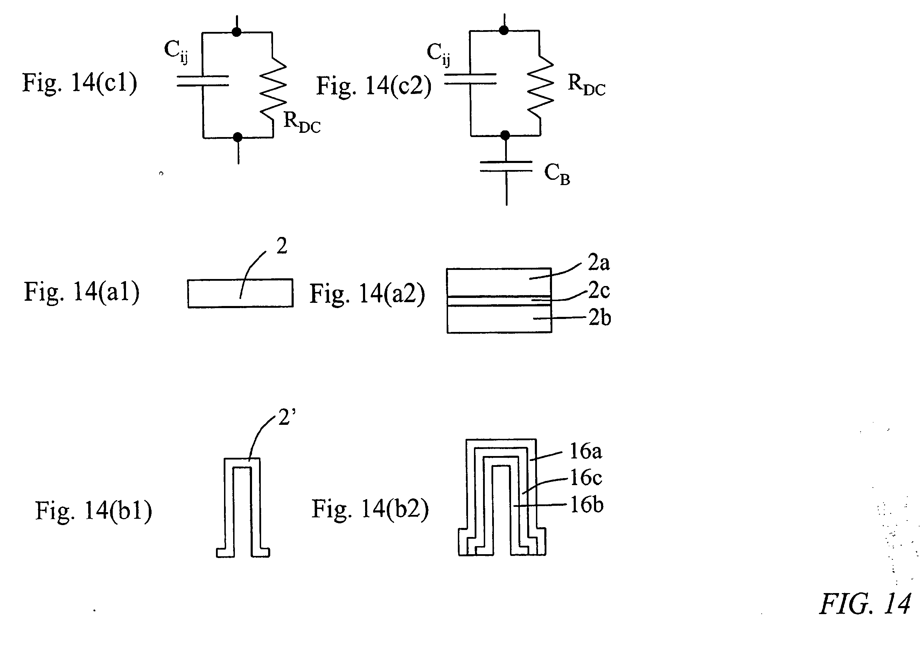

Referring to Figure 14, the electrode plate 2 of Figure 1 is shown schematically in Figure 14(a1) and the electrode plate 2' of Figure 12 is shown in Figure 14(b1). Both of the electrode plates 2, 2' may have a leaky resistive components as illustrated by the equivalent circuit shown in Figure 14(c1) with a DC leakage resistance $R_{DC}$. It may be desirable to suppress DC leakage through the resistance $R_{DC}$. This can be achieved as shown in Figures 14(a2) and 14(b2) for the plates 2, 2' respectively. The effect is to include an additional capacitor $C_B$ to block the DC component, as illustrated in the equivalent circuit of Figure 14(c2).

A suitable modification for the plate 2 shown in Figure 14(a2). The plate 2 is made of a composite comprising conductive plates 2A, 2B and a layer of insulator 2C between. The insulating layer 2C may comprise a coating e.g. of $SiO_2$ or SiN or $Ta_2O_5$.

A similar technique can be utilised for the electrode plate 2'of Figure 14(b2). In this arrangement, conductive layers 2'A, 2'B have an insulating coating 2'C provided between them.

*Example*

A DNA sensor constructed according to the design of Figures 11, 12 and 13 has an 8 x 8 array of wells 41, each plan $0.2\mu m$ x $5\mu m$ with depth 1 $\mu m$. Each circuit unit $U_{ij}$ has an associated capacitance $C_{ij}$ of 100 fF. Each of the circuit units is separately addressable as described with references to Figures 9 and 10. The capacitance of the individual circuit units $C_{ij}$ is of the order of 100 fF. The ON currents of the inverters $I_{ij}$ can be switched between currents of the order of $100\mu a$ to 0.01 $\mu a$ by changing the substrate voltage $V_{NS}$. In this example $V_{DD}$ = 1.5V and $V_{SS}$ = 0V.

For the selected circuit unit, the delay time is of the order of $15\mu s$. For the unselected circuit units, the delay time is of the order of 1.5ns x (8x8-1) = 94.5ns. It follows that each capacitance $C_{ij}$ can be detected within 1 %. The oscillation frequency is $1/15\mu s$ = 66.7kHz.

Many modifications and variations of the described sensor are possible. For example, the wells 41 may be configured as elongate trenches or other surface topographies may be employed. Also, the circuit units may be connected in oscillator circuits of different configurations.

**Claims**

1. A sensor comprising an array of capacitive elements (2, 2') configured to form a capacitive coupling ($C_{i,j}$) as a function of a medium (3, 5) to be sensed, a plurality of sensor circuit units ($U_{i,j}$) associated with the capacitive elements, the circuit units each exhibiting a respective time constant ($t_{i,j}$) as a function of the capacitive coupling of their associated capacitive element, **characterised by** the sensor circuit units being interconnected in series in a circuit that oscillates with a frequency (f) dependent on the time constants of the circuit units, and frequency detection circuitry (9) to detect the frequency (f) of oscillation of the circuit.

2. A sensor according to claim 1 wherein the circuit comprises a ring oscillator.

3. A sensor according to claim 1 or 2 wherein the capacitive elements comprise conductive members (2) for forming a capacitive coupling with the medium to be sensed.

4. A sensor according to claim 3 wherein the medium to be sensed comprises a solid body (3) and the members (2) are configured to form a capacitive coupling with the body when proximate thereto.

5. A sensor according to claim 3 wherein the medium to be sensed is fluid based (5), and the conductive members (2) are configured to form part of a capacitor of which the capacitance is a function of characteristics of the fluid.

6. A sensor according to claim 5 including a capacitor plate (4) spaced from the conductive members (2) to provide a region to receive the fluid.

7. A sensor according to claim 5 wherein the conductive members (2') are configured to form a mutual capacitive coupling with one another for sensing characteristics of the fluid.

8. A sensor according to any one of claims 3 to 5 wherein the conductive members comprise plates that are formed on a substrate (1) together with the circuit units.

9. A sensor according to any preceding claim including wells (41) to receive a fluid to be sensed, the conductive members (2') extending into the wells.

10. A sensor according to any one of claims 3 to 9 configured for use as a DNA sensor.

11. A sensor according to claim 10 wherein the conductive members have been functionalised for detecting predetermined DNA targets.

12. A sensor according to any preceding claim including selecting circuitry (35-40) to select the circuit units individually such that the frequency characteristic of the oscillatory circuit is mainly dependent on said capacitive coupling of the or each said selected circuit unit.

13. A sensor according to any preceding claim wherein the circuit units comprise inverters ($I_{i,j}$) with a time constant that is a function of said capacitive coupling.

14. A sensor according to claim 13 wherein the inverters each include an input to alter the time constant thereof selectively.

15. A sensor according to claim 13 or 14 wherein the inverters comprise complementary transistors (10, 11).

16. A sensor according to claim 13, 14 or 15 wherein the circuit units each include circuitry (38-40) configured to change the channel current of one of the transistors (11) so as to alter the time constant of the circuit unit.

17. A method of operating a sensor according to any preceding claim including placing a fluid based medium to be sensed in contact with the capacitive elements and monitoring the frequency of operation of the oscillatory circuit.

18. A method according to claim 17 including selecting one the circuit units and driving it such that it has a time constant greater than the others.

**Patentansprüche**

1. Sensor mit einer Anordnung von kapazitiven Elementen (2, 2'), die zum Bilden einer kapazitiven Kopplung ($C_{i,j}$) als Funktion eines zu erfassenden Mediums (3, 5) konfiguriert sind, und einer Vielzahl von Sensor-Schaltungseinheiten ($U_{i,j}$), die zu den kapazitiven Elementen gehören, wobei die Schaltungseinheiten jeweils eine jeweilige Zeitkonstante ($t_{i,j}$) als Funktion der kapazitiven Kopplung ihres zugehörigen kapazitiven Elements zeigen, **dadurch gekennzeichnet, dass** die Sensor-Schaltungseinheiten in einer Schaltung in Reihe geschaltet sind bzw. miteinander verbunden sind, die mit einer Frequenz (f) oszilliert, die von den Zeitkonstanten der Schaltungseinheiten abhängt, und eine Frequenzerfassungsschaltung (9) zum Erfassen der Frequenz (f) der Oszillation der Schaltung vorgesehen ist.

2. Sensor nach Anspruch 1, wobei die Schaltung einen Ringoszillator aufweist.

3. Sensor nach Anspruch 1 oder 2, wobei die kapazitiven Elemente leitende Elemente (2) zum Bilden einer kapazitiven Kopplung mit dem zu erfassenden Medium aufweisen.

4. Sensor nach Anspruch 3, wobei das zu erfassende Medium einen festen Körper (3) aufweist und die Elemente (2) konfiguriert sind, um eine kapazitive Kopplung mit dem Körper zu bilden, wenn sie diesem nahe sind.

5. Sensor nach Anspruch 3, wobei das zu erfassende Medium ein auf Fluid basierendes Medium (5) ist und die leitenden Elemente (2) konfiguriert sind, um einen Teil eines Kondensators zu bilden, von welchem die Kapazität eine Funktion von Charakteristiken des Fluids ist.

6. Sensor nach Anspruch 5, der eine Kondensatorplatte (4) enthält, die von den leitenden Elementen (2) beabstandet ist, um einen Bereich zum Empfangen des Fluids zur Verfügung zu stellen.

7. Sensor nach Anspruch 5, wobei die leitenden Elemente (2') konfiguriert sind, um eine wechselseitige kapazitive Kopplung miteinander zum Erfassen von Charakteristiken des Fluids zu bilden.

8. Sensor nach einem der Ansprüche 3 bis 5, wobei die leitenden Elemente Platten aufweisen, die auf einem Substrat (1) zusammen mit den Schaltungseinheiten ausgebildet sind.

**9.** Sensor nach einem der vorangehenden Ansprüche, der Wannen (41) enthält, um ein zu erfassendes Fluid aufzunehmen, wobei sich die leitenden Elemente (2') in die Wannen erstrecken.

**10.** Sensor nach einem der Ansprüche 3 bis 9, der zur Verwendung als DNA-Sensor konfiguriert ist.

**11.** Sensor nach Anspruch 10, wobei die leitenden Elemente zum Erfassen von vorbestimmten DNA-Zielen funktionalisiert worden sind.

**12.** Sensor nach einem der vorangehenden Ansprüche, der eine Auswahlschaltung (35 - 40) enthält, um die Schaltungseinheiten individuell so auszuwählen, dass die Frequenzcharakteristik der Oszillatorschaltung hauptsächlich von der kapazitiven Kopplung der oder jeder ausgewählten Schaltungseinheit abhängt.

**13.** Sensor nach einem der vorangehenden Ansprüche, wobei die Schaltungseinheiten Inverter ($I_{i,j}$) mit einer Zeitkonstanten aufweisen, die eine Funktion der kapazitiven Kopplung ist.

**14.** Sensor nach Anspruch 13, wobei die Inverter jeweils einen Eingang zum selektiven Ändern ihrer Zeitkonstanten enthalten.

**15.** Sensor nach Anspruch 13 oder 14, wobei die Inverter komplementäre Transistoren (10,11) aufweisen.

**16.** Sensor nach Anspruch 13, 14 oder 15, wobei die Schaltungseinheiten jeweils eine Schaltung (38 - 40) enthalten, die zum Ändern des Kanalstroms eines der Transistoren (11) konfiguriert ist, um die Zeitkonstante der Schaltungseinheit zu ändern.

**17.** Verfahren zum Betreiben eines Sensors nach einem der vorangehenden Ansprüche, das ein Anordnen eines zu erfassenden auf Fluid basierenden Mediums in Kontakt mit den kapazitiven Elementen und ein Überwachen der Betriebsfrequenz der Oszillatorschaltung enthält.

**18.** Verfahren nach Anspruch 17, das ein Auswählen einer der Schaltungseinheiten und ihr derartiges Antreiben, dass sie eine Zeitkonstante hat, die größer als die anderen ist, enthält.


**Revendications**

**1.** Capteur comprenant un groupement d'éléments capacitifs (2, 2') configuré pour former un couplage capacitif ($C_{i,j}$) en fonction d'un milieu (3, 5) à détecter, plusieurs unités ($U_{i,j}$) de circuit de capteur associées aux éléments capacitifs, les unités de circuit présentant chacune une constante de temps ($t_{i,j}$) respective en fonction du couplage capacitif de leur élément capacitif associé, **caractérisé en ce que** les unités de circuit de capteur sont interconnectées en série dans un circuit qui oscille avec une fréquence (f) dépendant des constantes de temps des unités de circuit, et des circuits (9) de détection de fréquences pour détecter la fréquence (f) d'oscillation du circuit.

**2.** Capteur selon la revendication 1, dans lequel le circuit comprend un oscillateur en anneau.

**3.** Capteur selon la revendication 1 ou 2, dans lequel les éléments capacitifs comprennent des éléments conducteurs (2) pour former un couplage capacitif avec le milieu à détecter.

**4.** Capteur selon la revendication 3, dans lequel le milieu à détecter comprend un corps solide (3) et dans lequel les éléments (2) sont configurés pour former un couplage capacitif avec le corps lorsqu'ils sont proches de celui-ci.

**5.** Capteur selon la revendication 3, dans lequel le milieu à détecter est à base de fluide (5), et les éléments conducteurs (2) sont configurés pour faire partie d'un condensateur dont la capacité est fonction des caractéristiques du fluide.

**6.** Capteur selon la revendication 5, incluant une plaque (4) de condensateur espacée des éléments conducteurs (2) pour donner une région propre à recevoir le fluide.

**7.** Capteur selon la revendication 5, dans lequel les éléments conducteurs (2') sont configurés pour former un couplage capacitif mutuel les uns avec les autres pour détecter les caractéristiques du fluide.

8. Capteur selon l'une quelconque des revendications 3 à 5, dans lequel les éléments conducteurs comprennent des plaques qui sont formées sur un substrat (1) conjointement avec les unités de circuit.

9. Capteur selon l'une quelconque des revendications précédentes, incluant des puits (41) pour recevoir un fluide à détecter, les éléments conducteurs (2') s'étendant dans les puits.

10. Capteur selon l'une quelconque des revendications 3 à 9, configuré pour utilisation comme un capteur d'ADN.

11. Capteur selon la revendication 10, dans lequel les éléments conducteurs ont été rendus propres à assurer la fonction de détection de cibles prédéterminées d'ADN.

12. Capteur selon l'une quelconque des revendications précédentes, incluant des circuits (35 à 40) de sélection pour sélectionner les unités de circuit individuellement de façon que la caractéristique de fréquence du circuit oscillant dépende principalement dudit couplage capacitif de ladite unité ou de chaque dite unité de circuit sélectionnée.

13. Capteur selon l'une quelconque des revendications précédentes, dans lequel les unités de circuit comprennent des inverseurs ($I_{i,j}$) avec une constante de temps qui est fonction dudit couplage capacitif.

14. Capteur selon la revendication 13, dans lequel les inverseurs comprennent chacun une entrée pour altérer sélectivement sa constante du temps.

15. Capteur selon la revendication 13 ou 14, dans lequel les inverseurs comprennent des transistors complémentaires (10, 11).

16. Capteur selon la revendication 13, 14 ou 15, dans lequel les unités de circuit comprennent chacune des circuits (38 à 40) configurés pour modifier le courant de canal de l'un des transistors (11) de façon à altérer la constante de temps de l'unité de circuit.

17. Procédé de mise en oeuvre d'un capteur selon l'une quelconque des revendications précédentes, incluant la mise en place d'un milieu à base de fluide à détecter en contact avec les éléments capacitifs et la surveillance de la fréquence de fonctionnement du circuit oscillant.

18. Procédé selon la revendication 17, incluant la sélection de l'une des unités de circuit et son pilotage de façon qu'elle ait une constante de temps plus grande que les autres.

*FIG. 1*

*FIG. 2*

Detection body

C   C   C   C   C

1   2

*FIG. 3*

Fixed plate

Detection material

4

5

1   2

FIG. 4

FIG. 5

EP 1 452 867 B1

FIG. 6

FIG. 7

*FIG. 8*

FIG. 9

EP 1 452 867 B1

*FIG. 10*

$41_{1,1}$ 2'

$41_{1,m}$ 2'

43  2'a  $41_{i,j}$  2'b  Detection material

42

45

44

1

$41_{i,j}$  2'

$41_{n,m}$ 2'

*FIG. 11*

*FIG. 12*

EP 1 452 867 B1

*FIG. 13*

$C_{1m}$ $C_{2m}$ $C_{nm}$

$C_{12}$ $C_{22}$ $C_{n2}$

$C_{11}$ $C_{21}$ $C_{n1}$

$U_{ij}$

Rst

6

7

3

8

9

detector

Fig. 14(c1)    $C_{ij}$    $R_{DC}$    Fig. 14(c2)    $C_{ij}$    $R_{DC}$    $C_B$

Fig. 14(a1)    2    Fig. 14(a2)    2a    2c    2b

Fig. 14(b1)    2'    Fig. 14(b2)    16a    16c    16b

*FIG. 14*